# FASCICULE DE BREVET EUROPEEN

(11) **EP 0 547 191 B1**
(45) Date de publication et mention de la délivrance du brevet: **04.12.1996**
(21) Numéro de dépôt: 92912288.5
(22) Date de dépôt: 05.06.1992
(51) Int. Cl.: A61K 9/50, A61K 9/51, A61K 9/54, A61K 47/42, A61K 47/48

(54) **VECTEUR PARTICULAIRE A TROPISME SELECTIF, PROCEDE DE PREPARATION ET COMPOSITION PHARMACEUTIQUE**
PARTIKELFÖRMIGES VEKTOR MIT SELEKTIVEM TROPISMUS, VERFAHREN ZUR HERSTELLUNG UND PHARMAZEUTISCHE ZUSAMMENSETZUNG
PARTICULATE VECTOR HAVING SELECTIVE TROPISM, METHOD OF PREPARATION AND PHARMACEUTICAL COMPOSITION

(30) Priorité: 05.06.1991 FR 9106812
(43) Date de publication de la demande: 23.06.1993
(73) Titulaire: BIOVECTOR THERAPEUTICS SA, F-31520 Ramonville-Saint-Agne (FR); CENTRE CLAUDIUS REGAUD, F-31520 Toulouse (FR)
(72) Inventeur: SAMAIN, Daniel, F-31400 Toulouse (FR); FAVRE, Gilles, F-31000 Toulouse (FR); NGUYEN, Frédérique, F-17640 Vaux-sur-Mer (FR); PEYROT, Marianne, F-46140 Douelle (FR); MERCIER, Philippe, F-31000 Toulouse (FR); SOULET, Nadine, F-31100 Toulouse (FR); DIRSON, Roselyne, F-31400 Toulouse (FR); CAZES, Sylvie Résidence le Gué de Noncesse, F-31130 Balma (FR); DE MIGUEL, Ignacio, F-31400 Toulouse (FR); MENIALI, Jaouad, F-31400 Toulouse (FR)
(74) Mandataire: Warcoin, Jacques
(86) Numéro de dépôt international: FR9200506
(87) Numéro de publication internationale: WO9221330

(56) Documents cités:
- EP-A- 0 344 040
- EP-A- 0 397 227

## Description

La présente invention se rapporte à un vecteur particulaire synthétique possédant une structure biomimétique calquée sur celle des lipoprotéines de basse densité (Low Density Lipoproteins ou LDL). Ce type de vecteur possède trois propriétés fondamentales :
1) c'est un système de délivrance intracellulaire de principe actif
2) il possède une capacité de ciblage de certains types de cellules en particulier des cellules tumorales ou des macrophages
3) c'est un système de vectorisation particulaire systémique capable de diffuser dans tout l'organisme.

Lors de l'administration systémique d'un médicament, la proportion atteignant effectivement la cible est souvent faible. Ceci est dû à la conjugaison de plusieurs facteurs :
1) les produits administrés ont fréquemment une stabilité limitée dans l'organisme et notamment dans la circulation sanguine. Ils sont donc dégradés ou complexés par des protéines avant d'atteindre leur cible et perdus pour l'effet thérapeutique souhaité.
2) ils ne sont pas toujours capables de franchir efficacement les membranes cellulaires. Ceci présente un inconvénient majeur dans le cas où le site d'action visé est situé à l'intérieur de la cellule puisqu'il va falloir maintenir une concentration plasmatique très élevée et sur une longue période pour pouvoir atteindre une concentration intracellulaire efficace.
3) la spécificité d'action de la molécule est généralement faible. Ceci se traduit à nouveau par la nécessité d'administrer de fortes doses de médicaments afin qu'une quantité suffisante atteigne le site d'action recherché pour l'activité thérapeutique. Il en résulte une activité en une multiplicité de sites de l'organisme et l'apparition d'effets secondaires indésirables.

C'est pourquoi il est particulièrement intéressant de disposer d'un moyen de protéger le médicament après son administration dans l'organisme et de lui permettre d'exercer son action à l'intérieur des cellules sur lesquelles on désire que la molécule agisse. Il serait ainsi possible d'augmenter de façon significative l'efficacité de molécules biologiquement actives à propriétés pharmacologiques, notamment de médicaments antiparasitaires, antimicrobiens, antiviraux ou anticancéreux.

### Ciblages des cellules cancéreuses par des médicaments cytotoxiques

L'utilisation en clinique humaine de médicaments cytotoxiques est en effet limitée par leur incapacité à distinguer les cellules normales des cellules tumorales. Cette incapacité et leur importante toxicité représentent un problème majeur du traitement des affections cancéreuses. Ainsi tout moyen permettant d'augmenter la fraction de principes actifs atteignant la masse tumorale est du plus grand intérêt. Parmi ces moyens, on peut évoquer l'incorporation de médicaments cytostatiques dans des macromolécules servant de "transporteurs" et choisies pour leur capacité de ciblage des cellules tumorales.

Malgré quelques résultats partiels encourageants, aucun de ces vecteurs ne semble, à ce jour, avoir fait la preuve d'une réelle efficacité en clinique humaine, et de nombreux problèmes restent à résoudre, notamment :
- le piégeage des transporteurs par le système réticulo-endothélial (SRE)
- le relargage du principe actif au niveau de la cible
- les réactions immunitaires vis-à-vis du vecteur

### Ciblage des macrophages par des médicaments antiparasitaires, antimicrobiens ou antiviraux

Il existe de nombreuses affections dans lesquelles l'agent pathogène se multiplie ou survit à l'état latent de façon intracellulaire dans les macrophages. Le rôle de ces derniers est normalement de phagocyter ces agents pathogènes et de les détruire. Cependant un certain nombre des microorganismes a évolué de façon à échapper aux systèmes lytiques des macrophages : secrétion d'inhibiteurs d'enzymes lytiques, inhibiteurs de radicaux libres, inhibition des enzymes lytiques par altération du pH intracellulaire, biosynthèse d'enveloppes résistants aux enzymes, etc... La localisation intracellulaire de ces microorganismes leur permet d'éviter d'être neutralisés par les anticorps circulants et d'être protégés de l'action des médicaments ne diffusant pas dans le milieu cellulaire. L'éradication de ces affections intracellulaires est donc très difficile avec des traitements très longs, peu efficaces et de nombreux cas de rechute.

### Délivrance intracellulaire de molécules biologiquement actives

Il existe un certain nombre de molécules qui possèdent une très grande spécificité d'action mais dont l'utilisation en tant que médicament est limitée soit par leur instabilité dans le milieu plasmatique soit par leur incapacité à diffuser dans le milieu cellulaire.

L'intérêt potentiel thérapeutique de ces molécules et leur spécificité d'action est facilement démontré et mis en évidence sur des modèles **in vitro**. Malheureusement cet intérêt n'est pas confirmé **in vivo** par suite des difficultés que nous avons évoquées précédemment. Il existe de très nombreux produits appartenant à cette catégorie, notamment des peptides, des protéines et des oligonucléotides.

Parmi les candidats les plus intéressants capables d'assurer l'ensemble de ces fonctions de transports, de délivrance et de ciblage, les lipoprotéines de basse densité (Low Density Lipoproteins ou LDL) se distinguent par une série de propriétés remarquables.

Les LDL, principaux transporteurs du cholestérol plasmatique, sont les lipoprotéines les plus sollicitées dans l'apport de cholestérol exogène aux cellules. Celles-ci, constituées d'un noyau apolaire de triglycérides et d'esters de cholestérol, comportent en périphérie une monocouche de phospholipides, de cholestérol libre et d'apoliprotéine B. Par leur taille de 20nm, celles-ci sont capables de diffuser de façon systémique dans tout l'organisme pour atteindre chaque cellule individuellement.

Les cellules incorporent les LDL en particulier par un mécanisme qui met en cause un récepteur membranaire spécifique de haute affinité. Après fixation au récepteur, plusieurs mécanismes autorégulés par la cellule aboutissent à une dégradation par des enzymes lysosomales des LDL ainsi internalisées, ce qui a pour effet de libérer le cholestérol directement utilisable pour la cellule.

L'utilisation de ce système de transport permet donc, à condition bien sûr de pouvoir incorporer des quantités suffisantes de médicament à l'intérieur des LDL, d'assurer une protection des principes actifs dans le milieu plasmatique et leur délivrance à l'intérieur du milieu cellulaire.

Par ailleurs, bien que pratiquement toutes les cellules expriment des récepteurs aux LDL, il existe des différentiels d'expression qu'il est possible d'utiliser et même dans certains cas de magnifier pour parvenir à assurer un ciblage par captation ou par décharge préférentielles. Les différentiels d'expression intéressants peuvent être négatifs ou positifs.

Par exemple, on peut citer le tissu cardiaque et le tissu rénal qui présentent une faible expression de récepteurs aux LDL, ce qui est très intéressant étant donné la susceptibilité de ces tissus à de nombreux médicaments. Bien que les cellules du système nerveux central présentent des récepteurs aux LDL, celles-ci ne sont pas capables de franchir la barrière hémato-méningée. La vectorisation par les LDL permet donc d'obtenir une décharge de ces trois organes.

Parmi les tissus présentant un différentiel d'expression de récepteur aux LDL positif, on peut mentionner, au niveau physiologique, le tissu hépatique et, au niveau physiopathologique, le tissu tumoral.

Dans ce dernier cas, il a été démontré que les cellules tumorales grâce à leur activité proliférative et l'altération de leur système de régulation du cholestérol présentent à la surface de leur membrane un plus grand nombre de récepteurs aux LDL que les cellules saines correspondantes et captent de ce fait davantage de LDL (voir par exemple Biochemica Biophysica Acta 1003 (1989) 301-306).

Des travaux ont été publiés sur l'utilisation des LDL comme vecteur d'agents cytotoxiques. Différentes techniques d'incorporation ont été décrites, du simple contact lipoprotéine/principe actif cytotoxique au greffage covalent sur l'apoB. Les complexes ainsi obtenus conservent leur capacité d'être spécifiquement reconnus par le récepteur membranaire de haute affinité et présentent un métabolisme **in vivo** chez la souris identique à celui des LDL natives.

Par ailleurs les LDL subissent à la longue une oxydation qui modifie leur reconnaissance. Les LDL oxydées ne sont plus reconnues par le récepteur cellulaire à l'apolipoprotéine B100/E mais par contre sont reconnues spécifiquement par un autre récepteur situé sur les macrophages. Il est donc possible grâce aux LDL oxydées de cibler spécifiquement le macrophage. Les propriétés de phagocytes du macrophage font qu'il n'est pas très difficile de vectoriser un principe actif vers les macrophages du SRE puisque n'importe quelle particule administrée par voie intraveineuse sera reconnue comme étrangère à l'organisme et phagocytée très rapidement. Toutefois les particules doivent posséder une taille suffisamment importante ( 100nm) pour pouvoir être phagocytées efficacement et ces tailles sont incompatibles avec une bonne diffusion systémique.

L'utilisation de particules de diamètre supérieur à 100nm permet donc de cibler les macrophages du SRE qui sont facilement accessibles mais pas les macrophages tissulaires. L'utilisation de LDL oxydées qui diffusent de manière systémique permet donc de cibler l'ensemble des macrophages.

L'utilisation de LDL natives présente cependant un certain nombre de limitations :
- le noyau des LDL est hautement apolaire et de ce fait il ne peut stocker que des médicaments eux-mêmes apolaires
- la capacité de stockage des noyaux est limitée par la nécessité de conserver leur intégrité structurale indispensable au métabolisme des LDL
- les LDL doivent être isolées du plasma humain et possèdent une conservation limitée

C'est pourquoi la présente invention se rapporte à un vecteur particulaire synthétique caractérisé en ce qu'il possèdent un tropisme sélectif pour des types particuliers de cellules et une délivrance intracellulaire et en ce qu'il comporte :
- un noyau hydrophile non liquide
- une première couche ou couronne de nature lipidique liée au noyau par des liaisons covalentes
- une seconde couche ou feuillet externe de phospholipides liée à la première couche par des interactions hydrophobes
- des protéines ayant une affinité pour les récepteurs se
trouvant sur la cellule à cibler, notamment des molécules d'apolipoprotéine B, greffées sur la couche de phospholipides.

Ce type de vecteur particulaire est capable d'encapsuler efficacement des principes actifs notamment des agents cytotoxiques de nature variée, tout en présentant les mêmes caractéristiques que les LDL, notamment leur taille et leur capacité à être reconnues et internalisées par les cellules et par exemple préférentiellement par les cellules tumorales.

Ces vecteurs sont construits à partir de particules de Bio Vecteurs Supra Moléculaires (BVSM) qui font l'objet des demandes de brevet EP 344 040 et FR 91 06743.

Ces BVSM ont une taille comprise entre 15 et 25nm.

Le noyau hydrophile non liquide sera essentiellement un polymère hydrophile, de préférence un polysaccharide biodégradable, choisi dans le groupe comprenant le dextrane, l'amidon et la cellulose et leurs dérivés.

Une première couche lipidique est liée au noyau polysaccharidique de 20nm de diamètre. Cette première couche est fixée par acylation régiosélective d'acides gras en périphérie des noyaux de polysaccharides réticulés.

La seconde couche ou feuillet externe est constituée de phospholipides identiques à ceux présents dans les LDL.

Le greffage de l'apolipoprotéine B est une étape délicate dans la synthèse de ces vecteurs. En effet l'apoB est une protéine hydrophobe et susceptible de s'agréger aisément par l'intermédiaire de ponts disulfures covalents. Cette agrégation est irréversible et dénature complètement la protéine qui n'est alors plus reconnue par le récepteur spécifique. Il faut donc isoler et greffer l'apoB dans des conditions très douces et non-dénaturantes. Nous avons choisi deux stratégies :
- isolement de l'apoB seule en présence de détergent : le détergent permet de solubiliser l'apoB en solution aqueuse dans des conditions non-dénaturantes et celle-ci est transférée des micelles de détergent sur les BVSM par dialyse du détergent
- isolement de l'apoB avec son environnement phospholipidique: l'apoB avec le feuillet phospholipidique des LDL, partiel ou complet, est greffée sur des BVSM plus ou moins riches en phospholipides

Ce type de vecteur peut servir au ciblage d'autres types de cellules moyennant de légères modifications.

En effet des modifications mineures de l'apoB changent le type de récepteur capable de reconnaître les LDL et le greffage de l'apoB modifiée à la surface de vecteurs permet de cibler spécifiquement d'autres types de cellules.

La présente invention concerne également un vecteur particulaire répondant à la définition ci-dessus et dans lequel les molécules d'apolipoprotéine B sont acétylées, oxydées et/ou ont subi tout type de modifications chimiques. Ce type de vecteur présente un tropisme sélectif pour les macrophages. Il trouvera une application particulièrement intéressante dans les affections impliquant ces cellules : infections virales, parasitoses, bactérioses intracellulaires.

La présente invention concerne un vecteur particulaire tel que défini précédemment et caractérisé en ce que le noyau hydrophile comporte en outre un principe actif.

En effet, pour ne pas perturber le mécanisme de reconnaissance cellulaire par une modification des propriétés du surface de la particule, les principes actifs sont chargés à l'intérieur du noyau polysaccharidique.

Selon le type de vecteur choisi, le principe actif sera dirigé vers un type de cellules plus particulièrement.

C'est ainsi que la présente invention concerne un vecteur particulaire tel que défini précédemment et caractérisé en ce que le principe actif est choisi de préférence dans le groupe comprenant des médicaments anticancéreux, réversants de résistance aux médicaments anticancéreux, immunomodulateurs, oligonucléotides, ARN messagers anti-sens, hépatoprotecteurs, inhibiteurs de HMGCoA réductase.

Selon l'un des aspects préférés de l'invention, le principe actif anticancéreux est un agent intercalant choisi dans le groupe des anthracyclines, et plus particulièrement parmi l'aclarubicine, l'épirubicine, la daunorubicine, la doxorubicine et la zorubicine.

Elle concerne également un vecteur particulaire tel que défini précédemment et caractérisé en ce que le principe actif est choisi dans le groupe comprenant antiviraux, antibactériens, antifongiques, antiparasitaires, antiSIDA.

Les principes actifs appartenant à ce dernier groupe seront de préférence encapsulés dans des vecteurs ayant un tropisme pour les macrophages, ces vecteurs portant des apolipoprotéines B acétylées et/ou oxydées.

La structure des vecteurs particulaires leur permet d'encapsuler pratiquement n'importe quelle molécule.

La présente invent ion concerne également un procédé de préparation d'un vecteur particulaire synthétique possédant un tropisme sélectif pour un type de cellules caractérisé en ce que :
a) on prépare des vecteurs comportant un noyau hydrophile, une première couche de lipides et une deuxième couche de phospholipides,
b) on encapsule un principe actif à l'intérieur du noyau hydrophile,
c) on greffe sur la couche phospholipidique des molécules d'apolipoprotéine B purifiée à partir de LDL.

La taille des vecteurs ainsi obtenus est de l'ordre de 20nm.

L'étape c) peut notamment être réalisée par la technique de dialyse de détergent à partir d'apolipoprotéine B solubilisée sous forme de micelles. Le détergent utilisé est de préférence choisi dans le groupe des détergents dialysables: désoxycholate de sodium, octyl-B, Dglucopyrannoside...

L'apolipoprotéine B est effectivement présente sur ces vecteurs et possède des propriétés physico-chimiques identiques sur ces vecteurs et sur les LDL.

Le métabolisme des vecteurs synthétiques selon l'invention a été suivi par marquage à l'iode radioactif, par immunofluorescence indirecte et par microscopie électronique de transmission.

On a ainsi pu démontrer que l'apoB est bien ancrée sur les vecteurs selon l'invention et qu'elle est bien reconnue en tant que telle par le récepteur à apoB de cellules-modèles. Le métabolisme des vecteurs selon l'invention est comparable à celui des LDL naturelles.

La présente invention se rapporte également à une composition pharmaceutique caractérisée en ce qu'elle comporte un vecteur particulaire synthétique à tropisme sélectif et un support acceptable pour son administration.

Les exemples qui suivent sont destinés à illustrer l'invention.

Dans ces exemples, on se référera aux figures suivantes :
- FIGURE 1 :: Visualisation en microscopie électronique de l'apoB sur les vecteurs particulaires par réaction de Thierry.
- FIGURE 2 :: Détection de l'apoB sur les vecteurs particulaires par des anticorps monoclonaux.
- FIGURE 3 :: Visualisation du métabolisme des vecteurs particulaires selon l'invention.

### EXEMPLE 1 : PREPARATION DES NOYAUX DE POLYSACCHARIDES RETICULES A PARTIR D'UN AMYLOPECTINE

Dans un réacteur de 51 muni d'un piège à soude, on mélange 500g d'amylopectine avec 31 de soude 2N. Lorsque la solution est homogène, on verse 12ml d'épichlorhydrine tout en agitant vigoureusement. Le mélange réactionnel est encore agité pendant 1h puis laissé reposer pendant une nuit.

On obtient un gel élastique et friable. Ce gel est mis en suspension dans 101 d'eau et neutralisé par de l'acide chlorhydrique concentré et broyé mécaniquement avec un appareil à hélice (type Waring Blendor) afin d'obtenir des particules de quelques dizaines de microns de diamètre.

Le gel obtenu est ensuite lavé par filtration sur büchner ou centrifugation et séché par lyophilisation.

### EXEMPLE 2 : PREPARATION DE NOYAUX DE POLYSACCHARIDES RETICULES ACIDES : FONCTIONNALISATION PAR L'ACIDE SUCCINIQUE

500g de gel polysaccharidique obtenu précédemment sont dispersés dans 51 d'eau distillée et le pH est ajusté à 8,5 par NaOH et maintenu à cette valeur par l'intermédiaire d'un pHstat. La température est maintenue au-dessous de 10°C. On ajoute séquentiellement alors 42g de monochlorure de l'acide succinique de façon à maintenir le pH autour de 8 et la température en-dessous de 10°C. Lorsque tout le chlorure est ajouté, la suspension est encore agitée pendant 1h en la laissant revenir à température ambiante. Le gel obtenu est lavé par filtration sur büchner ou par centrifugation puis séché par lyophilisation.

On obtient 500g de gel présentant un taux de substitution de 0,05 équivalent par sucre déterminé par titration.

Par suite de la répulsion existant entre les charges de même signe, les substituants ioniques sont répartis de façon homogène dans le volume du gel.

Le taux de substitution ionique peut être facilement ajusté en augmentant la quantité stoechiométrique de monochlorure de l'acide succinique par rapport à la matrice polysaccharidique.

| | | | |
|---|---|---|---|
| Rapport monochlorure/matrice PS | 0,25 | 0,33 | 1 |
| Taux de substitution | 0,13 | 0,17 | 0,48 |

### EXEMPLE 3 : OBTENTION DE NOYAUX POLYSACCHARIDIQUES SUCCINYLES DE 20nm DE DIAMETRE

500g de gel obtenu précédemment sont dispersés dans 51 d'eau distillée et homogénéisés au moyen d'un homogénéisateur Rannie 12-51 H. La pression d'homogénéisation est de 900 bars et le débit de 801/h.

On obtient une suspension fluide de nanoparticules de polysaccharides réticulés acides dont la taille, mesurée par un nanosizer Coulter N4MD, est centrée autour de 20nm. Les nanoparticules sont alors séchées à l'aide d'un atomiseur Büchi 190 en présence de 5% de NH₄HCO₃. La concentration des particules est de 5%, la température de l'air de séchage est de 200°C.

### EXEMPLE 4 : PREPARATION DE LA COURONNE LIPIDIQUE

100g de nanoparticules atomisées de diamètre moyen 20nm, obtenues précédemment, sont mis en suspension dans 500ml de dichlorométhane anhydre. On ajoute ensuite 50g de chlorure d'acide palmitique et 50g de carbonate de soude sec. L'ensemble est laissé sous forte agitation à reflux et à l'abri de l'humidité pendant 48h.

Le dichlorométhane est alors évaporé et les particules acylées sont lavées d'abord par un mélange acide acétique 2% / méthanol (10/90) puis 2 fois par du méthanol. Entre chaque lavage, les particules sont récupérées par centrifugation.

Les particules sont finalement obtenues à l'état sec par évaporation du méthanol à l'évaporateur rotatif sous pression réduite. Le taux de greffage d'acide gras sur les noyaux polysaccharidiques est déterminé par la méthode de Lauwerys et est de 10% en poids.

### EXEMPLE 5 : ETABLISSEMENT DU FEUILLET PHOSPHOLIPIDIQUE EXTERNE

1g de particules ioniques acylées obtenues précédemment (20nm / 0,05 ligand ionique par unité glucose / 10% d'acide palmitique) est dispersé dans 5ml d'éthanol. La suspension résultante est injectée à l'aide d'une seringue dans une suspension de liposomes unilamellaires réalisée à partir d'un gramme de phospholipides (lécithines de jaune d'oeuf purifiées-Sigma Chemical Company) et 100ml d'eau distillée. Les liposomes sont préparés par la méthode classique du ballon puis soniqués 1h au bain pour obtenir des liposomes unilamellaire.

### EXEMPLE 6 : PREPARATION DES NOYAUX DE POLYSACCHARIDES RETICULES A PARTIR D'AMIDON SOLUBLE

Dans un réacteur de 51 muni d'un piège à soude, on mélange 500g d'amidon soluble (Prolabo) avec 21 de soude 2N. Lorsque la solution est homogène, on verse 12ml d'epichlorhydrine tout en agitant vigoureusement. Le mélange réactionnel est agité pendant 1h et puis laissé reposé pendant une nuit.

On obtient un gel élastique qui est mis en suspension dans 101 d'eau et neutralisé par de l'acide chlorhydrique concentré. Ce gel est ensuite broyé mécaniquement avec un appareil à hélice (type Waring Blender) afin d'obtenir des particules de quelques dizaines de microns de diamètre.

Le gel obtenu est ensuite lavé par filtration sur büchner ou par centrifugation et séché par lyophilisation.

### EXEMPLE 7 : FONCTIONNALISATION PAR L'ACIDE PHOSPHORIQUE

500g du gel polysaccharidique obtenu selon l'exemple 6 sont suspendus dans 2 litres de soude 2M et refroidis à 0°C. On ajoute progressivement sous agitation 240g d'oxychlorure de phosphore (POC13, 1,55 mol) et 550ml de NaOH 10M de façon à ce que les deux réactifs soient ajoutés de façon simultanée. La température est maintenue en-dessous de 10°C. Après la fin de l'addition, le mélange est agité encore pendant deux heures en le laissant revenir à température ambiante. Le mélange est ensuite neutralisé par l'acide chlorhydrique, filtré sur büchner et lavé avec de l'eau distillée jusqu'à neutralité.

On obtient après lyophilisation 550g (rdt. 91%) de gel ionique. Le taux de greffage est déterminé par titration à la soude 0,1N avec la phénolphtaléine comme indicateur. On trouve 1,5 mEq de charges négatives par gramme de gel.

### EXEMPLE 8 : OBTENTION DE NOYAUX POLYSACCHARIDIQUES PHOSPHORYLES DE 20nm DE DIAMETRE

100g du gel phosphorylé obtenu selon l'exemple 7 sont dispersés dans 101 d'eau distillée et homogénéisés au moyen d'un homogénéisateur Rannie 12-51 H. La pression d'homogénéisation est de 900 bars et le débit de 80 l/h.

On obtient une suspension fluide de nanoparticules de polysaccharides réticulés acides dont la taille, mesurée par un nanosizer Coulter N4MD, est centrée autour de 20nm. Les nanoparticules sont alors séchées par lyophilisation en, présence de 50g/l de bicarbonate d'ammonium.

### EXEMPLE 9 : PREPARATION DE LA COURONNE LIPIDIQUE PAR LA METHODE DES ACYLATIONS MULTIPLES

10g de particules de 20nm obtenues selon l'exemple 8 sont dispersées dans 30ml de dichlorométhane. On ajoute 1,7g de chlorure d'acide palmitique. Un réfrigérant muni d'une garde au carbonate de potassium est adapté sur le flacon réactionnel et la réaction est agitée énergiquement à reflux pendant une nuit. Le dichlorométhane est ensuite évaporé avec un évaporateur rotatif et le résidu est lavé plusieurs fois par l'éthanol puis séché sous vide. On obtient 10,3g de particules acylées (rdt. 98%). Le taux d'acides gras greffés mesure après saponification des particules est de 5%.

10g des particules acylées obtenues sont remises en suspension sous forte agitation dans 1 litre d'eau. Une fois que la dispersion est homogène, les particules sont à nouveau lyophilisées en présence de bicarbonate d'ammonium à 50g/l.

Les particules sont dispersées dans 30ml de dichiorométhane et réacylées suivant le protocole décrit précédemment. On obtient, après lavage, 9,5g de particules (rdt. 94%) avec un taux d'acides gras mesuré à 6%.

Un troisième cycle réactionnel, comprenant toutes les étapes déjà décrites (hydratation, lyophilisation, réacylation) conduit a 9g de particules acylées (rdt. total 88%) avec un taux d'acide gras de 6,5%.

### EXEMPLE 10 :ETABLISSEMENT DU FEUILLET PHOSPHOLIPIDIQUE SUR DES COEURS ACYLES DE 20nm PAR LA METHODE DE DIALYSE DE DETERGENT

La composition du feuillet phospholipidique utilisé est similaire a celle des LDL et est la suivante: (mélange type LDL)
- 53% de lécitihines de jaune d'oeuf purifiées (EYPC)
- 20% de sphingomyéline (SM)
- 4% de phosphatidylsérine (PS)
- 3% de lysophosphatidylcholine (LPC)
- 20% de cholestérol

### (Sigma Chemical Company)

Les lipides sont mélangés à l'état sec avec le détergent (53mg d'EYPC, 20mg de SM, 20mg de cholestérol, 4Mg de PS, 3mg de LPC et 292mg d'octyl-B, Dglucopyrannoside) et solubilisés dans 2ml de chloroforme. Le solvant est éliminé par évaporation rotative pour obtenir un film uniforme. Les lipides et le détergent sont ensuite hydratés peu à peu à l'aide d'une solution d'EDTA 1mM, pH=7 (20 Ml) sous agitation magnétique. La suspension obtenue est alors soumise aux ultra-sons dans un bain à 45°C pendant 15mn sous atmosphère inerte.

Les coeurs acylés (10mg) préparés selon l'exemple 9, dispersés dans 2ml d'octyl-B, D-glucopyrannoside sont mélangés aux lipides (7,5mg/1,5ml) sous ultra-sons puis dilués brutalement sous la CMC par injection dans 16ml d'EDTA 1mM, pH7. Le détergent est ensuite éliminé par 48h de dialyse contre une solution d'EDTA 1mM, pH=7.

### EXEMPLE 11 :INCORPORATION DANS LES BVSM, D'UN AGENT ANTIBACTERIEN : LA BUTIROSINE

La butirosine est un antibiotique de la famille des aminoglycosides. C'est une molécule constituée d'un aminocylitol lié par des liaisons glycosidiques à des sucres amines. Il s'agit d'un produit très polaire et basique soluble uniquement dans l'eau. Son poids moléculaire est de 556.
**a)** On prépare d'abord des noyaux acylés acides de 20nm caractérisés par un taux de greffage ionique correspondant à un acide succinique pour 5 sucres et préparés selon l'exemple 4. La couronne lipidique est constituée d'acide palmitique avec un taux de 10% par rapport à la matrice polysaccharidique.
   On mélange à l'état sec 1g de noyaux acylés et 0,5g de butirosine base (Park Davis). Le mélange est ensuite hydraté progressivement par ajout d'eau distillée. Le mélange est maintenu constamment agité et à 50°C. On ajoute ainsi 10ml d'eau en laissant le mélange revenir à température ambiante et on laisse encore 2h.
   La suspension est ensuite lyophilisée. Le résidu sec est dispersé dans 5ml d'éthanol et ajouté à une suspension de liposomes unilamellaires (1g de lécithines de jaune d'oeuf purifiées dans 50ml d'eau distillée). Après sonication au bain pendant 1h, la suspension est ultrafiltrée et la butirosine libre présente dans l'ultrafiltrat est dosée par HPLC. Les résultats indiquent la présence de 25mg de butirosine dans l'ultrafiltrat, ce qui correspond à un rendement d'incorporation de 95% et u taux d'incorporation de 47,5% en poids par rapport au coeur acylé.
**b)** Dans un premier temps, des noyaux acylés acides de 20nm sont préparés selon l'exemple 9. On mélange ensuite, 50mg de noyaux acylés avec 25mg de butirosine base (Park-Davis) dilués dans 1ml d'eau distillée. Le mélange est maintenu constamment agité à température ambiante, pendant une nuit.
   La suspension obtenue est dispersée en présence d'OctylbétaD-Glucopyranoside (OGP) (Fluka) jusqu'à une molarité finale de 50mM et est ajoutée goutte à goutte à une solution de phospholipides (50mg d'un mélange de lécithines de jaune d'oeuf purifiées / cholestérol (80/20) w/w, dispersés dans 10ml d'OGP 50mM). Après sonication au bain pendant 10 minutes, cette solution est brutalement diluée sous Ultra-Sons jusqu'à une molarité de 5mM en OGP puis, ultrafiltrée (point de coupure: 30 000 daltons). Les BVSM obtenus sont alors stérilisés sur filtres 0,22µm. L'analyse de taille effectuée au nanosizer (Coulter N4 SD) indique que 99% de ces BVSM ont un diamètre de 20nm (+/-2nm).
   La butirosine libre présente dans le filtrat est dosée par microbiologie. La concentration de l'antibiotique est déterminée par la mesure de l'aire d'inhibition de la croissance de Bacillus subtilis (ATCC 6633). Les résultats indiquent la présence de 2,5mg de butirosine libre dans l'ultrafiltrat, ce qui correspond à un rendement d'incorporation de 90% de butirosine et à un taux d'incorporation de 45% en poids de butirosine par rapport au poids de coeurs acylés.
   Les BVSM ainsi obtenus sont stérilisés par filtration sur 0,2µm et présentent un rendement de filtration de 95%. L'analyse de la taille est réalisée à l'aide d'un nanosizer (Coulter N4SD) et donne une répartition de 95% à 20nm+/-3nm.

### EXEMPLE 12 : INCORPORATION D'UN AGENT ANTI-CANCEREUX, LA DOXORUBICINE, DANS LES BVSM

La doxorubicine (Adriamycine ®) est un antibiotique anticancéreux appartenant à la famille des anthracyclines. C'est un produit amphiphile caractérisé par un aglycone polyaromatique conférant à la molécule des propriétés de fluorescence caractéristiques et par un sucre aminé, la daunosamine. Le poids moléculaire du chlorhydrate de doxorubicine est de 580.
**a)** On utilise les noyaux acylés succinylés avec l'acide succinique sous forme de sel de sodium préparés selon l'exemple 4.
   On mélange à l'état sec 1g de noyaux acylés et 0,7g de chlorhydrate de doxorubicine (Sigma Chemical Company). Le mélange est ensuite hydraté progressivement par 10ml d'eau distillée sous agitation et à température ambiante. Il est ensuite agité pendant 2h à température ambiante.
   La suspension obtenue est lyophilisée. Le résidu sec est dispersé dans 5ml d'éthanol et ajouté à une suspension de liposomes unilamellaires (3g de lécithines de jaune d'oeuf purifiées dans 50ml d'eau distillée). Après sonication au bain pendant 1h, la suspension est ultrafiltrée et la doxorubicine libre présente dans l'ultrafiltrat est dosée par HPLC. Les résultats indiquent la présence de 28mg de doxorubicine dans l'ultrafiltrat, ce qui correspond à un rendement d'encapsulation de 96% et un taux d'encapsulation de 67% en poids par rapport aux noyaux acylés.
**b)** On utilise les noyaux acylés décrits dans l'exemple 9.
   On disperse 0,5g de noyaux acylés dans 10ml d'éthanol. 0,20g de doxorubicine (Sigma Chemical Company) en solution aqueuse est ajoutée progressivement à la suspension de noyaux acylés sous agitation. Le mélange obtenu est ensuite agité pendant 2h à température ambiante et à l'abri de la lumière.
   De l'éthanol est ajouté à la suspension de noyaux acylés ainsi incorporés pour obtenir un taux de 75% en volume. Les noyaux acylés sont récupérés par centrifugation (10 000g-15min) et séchés. La doxorubicine libre est dosée par HPLC et les résultats indiquent la présence de 4mg de doxorubicine dans le surnageant. Le taux de chargement de la doxorubicine dans les coeurs acylés est de 39% et le rendement en doxorubicine est de 98%.
   Le résidu sec est repris par 175ml d'octyl-B, Dglucopyrannoside (OGP-Sigma Chemical Company) 50mM contenant 0,375g de phospholipides type LDL et dispersé au bain à ultra-sons. La suspension obtenue est ensuite injectée sous la sonde à ultra-sons dans 700ml d'eau distillée pour descendre à 10mM en OGP sous la CMC. Les BVSM chargés en doxorubicine ainsi formés sont dialysés extensivement pour éliminer le détergent. Ils sont ensuite stérilisés par filtration sur 0,2µm.
   La doxorubicine incorporée est dosée par HPLC après relargage dans une solution de NaH₂PO₄ 2M / éthanol - 70/30. Les résultats indiquent la présence de 0,17g de doxorubicine incorporée dans les BVSM et correspondent à un taux de chargement de 34% par rapport aux noyaux acylés et un rendement en doxorubicine de 85%.
   Les BVSM ainsi incorporés en doxorubicine ont été analysés en perméation de gel sur colonne TSK G6000PW pour évaluer leur taille. Le profil chromatographique de ces BVSM montre que leur taille est la même que celle des BVSM sans doxorubicine présentant une répartition de taille de 95% à 20nm+/-3nm.

### EXEMPLE 13 : INCORPORATION D'UN AGENT ANTIVIRAL, LA ZIDOVUDINE, DANS LES BVSM

La zidovudine ou azido-3 désoxythymidine (AZT) possède un caractère basique par son noyau thymidine. C'est un inhibiteur de la transcriptase inverse et utilisé comme agent antiviral, notamment dans le traitement du SIDA. Son poids moléculaire est de 267.
**a)** On utilise les mêmes noyaux que dans l'exemple 11 a).
   On mélange à l'état sec 1g de noyaux acylés et 0,33g de zidovudine (Sigma Chemical Company). Le mélange est ensuite hydraté progressivement par 10ml d'eau distillée sous agitation et à température ambiante. Il est ensuite agité pendant 2h à température ambiante.
   La suspension obtenue est lyophilisée. Le résidu sec est dispersé dans de l'éthanol et ajoutée à une suspension de liposomes unilamellaires (3g de lécithines de jaune d'oeuf purifiées dans 50ml d'eau distillée). Après 1h de sonication au bain, la suspension est ultrafiltrée et la zidovudine libre présente dans l'ultrafiltrat est dosée par HPLC. Les résultats indiquent la présence de 30mg de zidovudine dans l'ultrafiltrat, ce qui correspond à un rendement d'encapsulation de 91% et un taux d'encapsulation de 30% en poids par rapport aux noyaux acylés.
**b)** On utilise les noyaux acylés selon l'exemple 9.
   On disperse 0,5g de noyaux acylés dans 10ml d'éthanol. 0,150g de zidovudine (Sigma Chemical Company) en solution aqueuse est ajouté progressivement à la suspension de noyaux acylés sous agitation. Le mélange obtenu est ensuite agité pendant 2h à température ambiante.
   De l'éthanol est ajouté à la suspension de noyaux acylés ainsi incorporés pour obtenir un taux de 75% en volume. Les noyaux acylés sont récupérés par centrifugation (10 00g-15min) et séchés. La zidovudine libre est dosée par HPCL et les résultats indiquent la présence de 15mg dans le surnageant. Le taux de chargement de la zidovudine dans les coeurs acylés est donc de 27% et le rendement en zidovudine est de 95%.
   Le résidu sec est repris par 175ml d'octyl-B, Dglucopyrannoside (OGP-Sigma Chemical Company) 50mM contenant 0,375g de phospholipides type LDL et dispersé au bain à ultra-sons. La suspension obtenue est ensuite injectée sous la sonde à ultra-sons dans 700ml d'eau distillée pour descendre à 10mM en OGP. Les BVSM chargés en zidovudine ainsi formés sont dialysés extensivement pour éliminer le détergent. Ils sont ensuite filtrés sur membrane 0,2µm de manière stérile.
   La zidovudine incorporée est dosée par HPLC après relargage dans une solution de NaH₂PO₄ 2M / éthanol - 70/30. Les résultats indiquent la présence de 0,128g de zidovudine incorporée dans les BVSM et correspondent à un taux de chargement de 26% par rapport aux noyaux acylés et un rendement en zidovudine de 85%.

### EXEMPLE 14 :INCORPORATION D'UN AGENT ANTIMYCOBACTERIEN, L'ISONIAZIDE, DANS LES BVSM

L'isoniazide est le dérivé hydrazide de l'acide isonicotinique, ce qui lui confère un caractère basique. Il est très efficace dans le traitement de la tuberculose. Son poids moléculaire est de 137.
**a)** Les noyaux utilisés sont ceux décrits dans l'exemple 11 a).
   On mélange à l'état sec 1g de noyaux acylés et 0,17g d'isoniazide. Le mélange est ensuite hydraté progressivement par 10ml d'eau distillé à 40°C et agité à cette température et pendant 1h. On laisse le mélange revenir à température ambiante sous agitation pendant 1h.
   La suspension est ensuite lyophilisée. Le résidu sec est dispersé dans de l'éthanol et ajouté à une suspension de liposomes unilamellaires (3g de lécithines de jaune d'oeuf purifiées dans 50ml d'eau). Après sonication au bain pendant 1h, la suspension est ultrafiltré et l'isoniazide libre présent dans l'ultrafiltrat est dosé par HPLC. Les résultats indiquent la présence de 15mg d'isoniazide dans l'ultrafiltrat, ce qui correspond à un rendement de 91% et un taux d'incorporation de 15,5% en poids par rapport aux noyaux acylés.
**b)** Les noyaux utilisés sont les mêmes que ceux décrits dans l'exemple 9.
   On disperse 0,5g de noyaux acylés dans 10ml d'éthanol. 0,10g d'isoniazide (Sigma Chemical Company) en solution aqueuse est ajouté progressivement à la suspension de noyaux acylés sous agitation. Le mélange obtenu est ensuite agité pendant 1h à température ambiante.
   De l'éthanol est ajouté à la suspension de noyaux acylés ainsi incorporés pour obtenir un taux de 75% en volume. Les noyaux acylés sont récupérés par centrifugation (10 000g - 15min) et séchés. L'isoniazide libre est dosée par HPLC et les résultats indiquent la présence de 4mg dans le surnageant. Le taux de chargement d'isoniazide dans les coeurs acylés est de 19% par rapport aux noyaux acylés et un rendement de 96%.
   Le résidu sec est repris par 175ml d'octyl-B, D-glucopyronnoside (OGP Sigma Chemical Company) 50mM contenant 0,375g de phospholipides type LDL et dispersé au bain à ultra-sons. La suspension obtenue est ensuite injectée sous la sonde à ultra-sons dans 700ml d'eau distillée pour descendre à 10mM en OGP. Les BVSM chargés en isoniazide ainsi formés sont dialysés extensivement pour éliminer le détergent.
   L'isoniazide incorporée est dosée par HPLC après relargage dans une solution de NaH₂PO₄ 2M / éthanol - 70/30. Les résultats indiquent la présence de 0,087g d'isoniazide incorporée dans les BVSM et correspondent à un taux de chargement de 17% par rapport aux noyaux acylés et un rendement en isoniazide de 87%.

### EXEMPLE 15 :GREFFACE DE L'apoB SUR LES BVSM PAR LA METHODE DE DIALYSE DE DETERGENT

Les LDL utilisées sont extraites à partir de plasma sanguin de donneurs sains par ultra-centrifugation séquentielle en gradient de KBr.

Les BVSM utilisés sont synthétisés selon le protocole décrit précédemment à l'exemple 5 et possèdent les caractéristiques suivantes : coeur polysaccharidique acide à 0,05 ligand ionique par unité glucose, 10% d'acide palmitique, un feuillet phospholipidique qui est composé de 75% de dimyristoyl phosphatidyl choline et de 25% de sphingomyeline (% en poids) plus 10% de cholestérol (% par rapport au poids de phospholipides) pour les BVSM complets ou de lécithines de jaune d'oeuf pour les BVSM déficients en phospholipides. La taille des BVSM est de 20nm et le rapport coeur acylé/phospholipides est de 1/3 en poids.

Les quantités de LDL sont données en milligramme d'apoB dosée par turbidimétrie (croissance de la densité optique à 500nm due à la précipitation de l'apoB par un anticorps anti-apoB).

Ces données sont valables pour tous les exemples de greffage de l'apoB sur les BVSM.

Les LDL (3mg/0,5ml), préalablement dialysées contre une solution de tampon A (Na₂CO₃ 50 mM, pH=10), sont déstructurées par du désoxycholate de sodium (NaDC) (165mg) dans les proportions suivantes : 55 fois plus en poids que l'apoB et en ajustant le volume final pour obtenir une concentration finale en NaDC de 180mg/ml. La solution est vortexée pendant 1min puis agitée doucement pendant 1h à température ambiante. Le NaDC permet de déstructurer entièrement les LDL et de solubiliser l'apoB par l'intermédiaire de micelles de détergent dans des conditions non-dénaturantes.

Les différents constituants des LDL sont ensuite sépares selon leur poids moléculaire par gel-filtration (système FPLC de Pharmacia, colonne Superose 6 1,5cm x 30cm, phase mobile tampon B : tampon A + NaDC 10mM, débit 25ml/h) et collectés par fraction de 1ml. L'apoB se trouve sous forme de micelles mixtes de détergent, ce qui augmente son poids moléculaire apparent. Aussi elle est éluée dans le volume mort de la colonne. Les fractions contenant l'apoB sont déterminées par dosage des protéines puis rassemblées. Le rendement d'extraction de l'apoB est de 95% à 100%.

La solution de micelles d'apoB et de NaDC est ajoutée goutte-a-goutte à la suspension de BVSM (19,3mg/2ml dont 4,5mg de coeurs acylés et 13,5mg de DMPC et de sphingomyeline(75%-25%)) et 0,9 mg de cholestérol) mise en dialyse contre du tampon A. La suspension obtenue est ensuite dialysée contre du tampon A pendant 48h pour éliminer tout le NaDC.

La suspension obtenue est centrifugée à 5000g pendant 15min. Les BVSM-apoB (2,6mg d'apoB et 4,0mg de coeurs acylés) sont récupérés dans le surnageant.

Le désoxycholate de sodium peut être remplacé par tout autre détergent dialysable.

### EXEMPLE 16 : GREFFAGE DE L'apoB PAR ELIMINATION DE DETERGENT EN DEUX ETAPES

Cet exemple est une variante de l'exemple 1 où le détergent est éliminé en grande partie par perméation de gel puis par dialyse. Les BVSM utilisés sont les mêmes que précédemment.

Les LDL (3mg/0,5ml), préalablement dialysées contre une solution de tampon A (Na₂CO₃ 50m M, NaCl 50 mM, pH=10), sont déstructurées par du désoxycholate de sodium (NaDC) (165mg) dans les proportions suivantes: 55 fois plus en poids que l'apoB et en ajustant le volume final pour obtenir une concentration finale en NaDC de 180mg/ml. La solution est vortexée pendant 1min puis agitée doucement pendant 1 h à température ambiante.

Les différents constituants des LDL sont ensuite séparés selon leur poids moléculaire par gel-filtration (système Pharmacia, colonne Sepharose CL-4B 1, 6cm x 60cm, phase mobile tampon B) et collectés par frction de 1ml. Les fractions contenant l'apoB sont déterminées par dosage des protéines puis rassemblées.. L'excès de NaDC est ensuite éliminé par perméation de gel sur une colonne de Sephadex G-75 (1,6cm x30cm), équilibré et éluée par du tampon Tris-HCl 10mM à pH 9, pour obtenir l'apoB avec le minimum de NaDC nécessaire à la solubiliser en solution aqueuse de manière non dénaturante.

La solution d'apoB est ajoutée à une suspension de BVSM (19,3mg/2ml) puis soniquée au bain pendant 15min à 37°C. La suspension obtenue est dialysée pendant 48h contre du tampon A pour éliminer le NaDC.

La suspension obtenue est centrifugée à 5000g pendant 15min. Les BVSM-apoB (2,8mg d'apoB et 4,0mg de coeurs acylés) sont récupérés dans le surnageant.

### EXEMPLE 17 :GREFFACE DE L'apoB AVEC SON ENVIRONNEMENT PHOSPHOLIPIDIQUE SUR LES BVSM APRES EXTRACTION ORGANIQUE DU COEUR HYDROPHOBE DES LDL

Les LDL (2mg d'apoB/3ml), préalablement dialysées contre une solution d'EDTA à 3mM ajustée à pH=8, sont lyophilisées en présence de lactose (100mg) pendant 18h. Le lactose évite l'agrégation des LDL et la dénaturation de l'apoB.

Le lyophilisat est dispersé grossièrement dans de l'heptane (5ml) sous agitation magnétique puis centrifugé à 1000g pendant 10min. Le surnageant contenant les lipides du coeur hydrophobe des LDL est éliminé et le culot est extrait encore 2 fois dans les mêmes conditions. L'heptane permet d'extraire sélectivement les esters de cholestérol et les triglycérides sans enlever les phospholipides. Le lyophilisat ainsi qui contient l'apoB entourée du feuillet phospholipidique des LDL est ensuite séché sous courant d'azote.

Les coeurs acylés des BVSM en suspension dans de l'éther éthylique (3mg/1,5ml) sont ajoutés au lyophilisat et agités doucement à température ambiante pendant 1h. L'éther éthylique est ensuite évaporé à sec sous courant d'azote puis sous vide.

La poudre ainsi obtenue est réhydratée sous agitation à 37°C avec 2ml de tampon Tris-Glycine 10mM à pH=8 et soniquée pendant 3min au bain à ultra-sons. L'apoB est ainsi greffée en même temps que le feuillet phospholipidique sur les coeurs acylés. La suspension obtenue est homogénéisée sous agitation magnétique pendant 12h à 4°C puis dialysée contre du tampon (NaCl 150mM, Tris 50mM, EDTA 4%, pH=8) pour éliminer le lactose.

La suspension obtenue est centrifugée à 5000g pendant 15min. Les BVSM-apoB (1,6mg d'apoB et 2,5mg de coeurs acylés) sont récupérés dans le surnageant.

Le lactose utilisé dans cet exemple peut être substitué par le saccharose, le glucose ou tout autre sucre. Tout solvant organique solubilisant les lipides du coeur hydrophobe des LDL peut convenir à la place de l'heptane : hexane,...

### EXEMPLE 18 : GREFFAGE DE L'apoB AVEC SON ENVIRONNEMENT PHOSPHOLIPIDIQUE SUR LES BVSM APRES HYDROLYSE ENZYMATIQUE PARTIELLE DES LDL

Les LDL (2mg), préalablement dialysées contre du tampon I (K₂HPO₄-KH₂PO₄ 100mM, pH=7,4) sont mises en présence de stérol-ester hydrolase (EC3.1.1.13 de Pseudomonas **fluorescens**) (2 unités) et de sérum albumine bovine délipidée (2 à 10mg). La solution est incubée pendant 2h à 37°C sous agitation. L'enzyme, en hydrolysant les esters de cholestérol, déstructure légèrement les LDL et l'albumine capte les lipides ainsi libérés. Dans ces conditions, l'apoB est isolée de manière non-dénaturante avec son environnement phospholipidique qui la protège.

L'apoB ainsi isolée est lavée 3 fois avec du tampon I (20ml) par ultra-filtration (système Amicon) sur membrane de seuil de coupure de 300 000 en poids moléculaire pour éliminer l'enzyme et l'albumine chargée en lipides.

L'apoB obtenue est ajoutée sous ultra-sons à la suspension de BVSM, synthétisés avec un défaut de 20% en phospholipides, et soniquée pendant 15min à 37°C. La suspension obtenue est centrifugée à 5000g pendant 15min. Les BVSM-apoB (1,8mg d'apoB et 2,5mg de coeurs acylés) sont récupérés dans le surnageant.

### EXEMPLE 19 : CARACTERISATION DES BVSM-apoB

Pour chacune des analyses suivantes, nous avons réalisé des tests avec des LDL pour vérifier que les BVSM-apoB ont le même comportement que les LDL et avec des BVSM comme témoin négatif.

### Détection de l'apoB sur les BVSM-apoB

### -méthode cytochimique : visualisation des polysaccharides par la méthode de Thierry

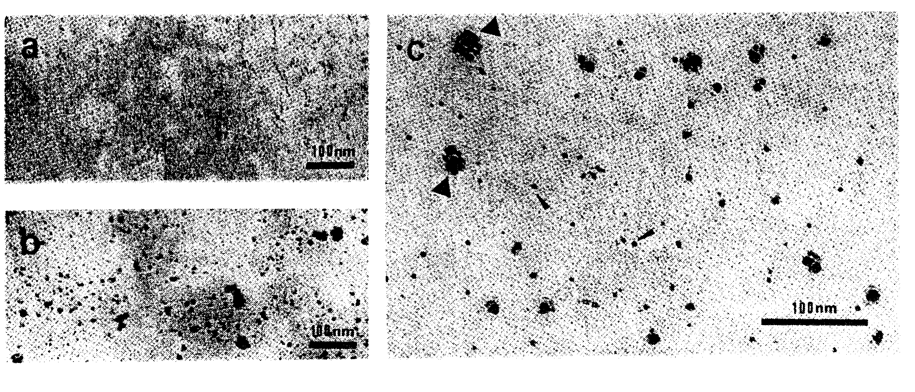

L'apoB et les BVSM sont détectés par la réaction de Thierry qui met en évidence les sucres (oxydation périodique des sucres, condensation avec de la thiocarbohydrazide et complexation par le protéinate d'argent) : l'apoB est glycosilée et les BVSM possèdent un coeur polysaccharidique. L'observation en microscopie électronique de transmission permet de visualiser l'apoB et les coeurs polysaccharidiques (figure 1).

Les LDL ou les BVSM-apoB sont déposées sur une grille de microscopie électronique recouverte en or. La grille est ensuite déposée pendant 45min sur des gouttes d'acide périodique 1% fraichement préparé. Après avoir été rincée 3 fois 10min avec de l'eau distillée, la grille est mise à l'obscurité dans des salières contenant de la thiosemicarbazide 1% dans de l'acide acétique 10%. Après 66 ou 72h, la grille est lavee par des bains successifs d'acide acétique de 10-5-2,5-1% puis par 3 bains d'eau distillée. Ensuite les particules sont colorees avec du protéinate d'argent à 1% dans l'eau pendant 30min à l'obscurité et enfin rincées 3 fois 5min avec de l'eau. La grille est ensuite observée en microscopie électronique de transmission.

### -méthode immunocytochimique

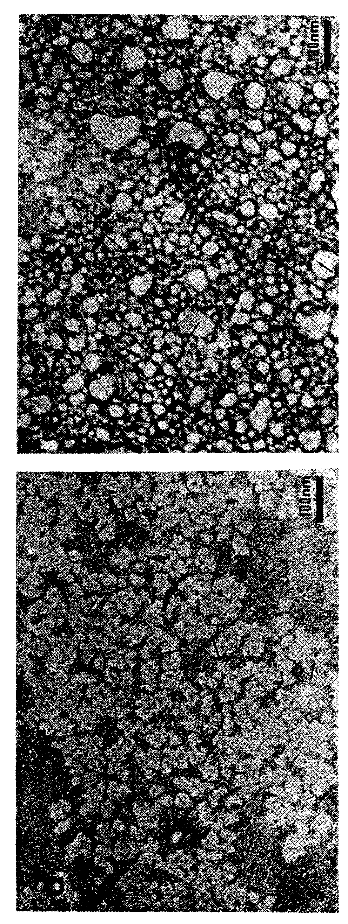

L'apoB est détectée par un anticorps polyclonal de chèvre anti-apoB et le complexe formé est révélé par la protéine G qui est couplée à un grain d'or de 5nm. L'observation en microscopie électronique de transmission met en évidence l'apoB sur les BVSM-apoB, les BVSM seuls donnant une réponse négative. (figure 2).

Les LDL ou les BVSM-apoB sont déposés sur une grille de microscopie électronique recouverte d'un support en nickel puis lavés 3 fois 5min à l'eau distillée. La grille est ensuite incubée 1h à température ambiante en présence d'anticorps polyclonal anti-apoB de chèvre (Tebu) qui est dilué au 1/200ième à partir de la solution-mère à 8mg/ml. La grille est ensuite rincée 6 fois 5min avec un mélange PBS + 1% BSA. Pour révéler l'anticorps fixé, nous utilisons la protéine G diluée au 1/20ième à laquelle des particules d'or de 5nm sont absorbées (Janssen). Après 3 bains de 5min dans du PBS + 1% BSA, dans du PBS seul puis de l'eau distillée, les particules sont observées au microscope électronique.

### -mesure de la taille des BVSM-apoB

L'observation en microscopie électronique de transmission et l'analyseur de particules submicroniques par diffusion de la lumière (Nanosizer Coulter N4MD) donnent une répartition en taille à plus de 95% à 20nm pour les BVSM-apoB.

### -profil électrophorétique des BVSM-apoB

L'électrophorèse est réalisée sur gel de polyacrylamide-SDS 4%. L'apoB greffée sur les BVSM et celle des LDL présentent la même migration électrophorétique. Celle-ci est conforme à la connaissance du poids moléculaire apparent de l'apoB. Aucune agrégation et aucun fragments de l'apoB n'apparaissent.

### -détermination de la densité des BVSM-apoB

La densité des BVSM-apoB est déterminée par ultra-centrifugation en gradient discontinu de KBr.

Les suspensions de LDL ou de BVSM-apoB (1mg/1mg/1,5ml) dont la densité est ajustée à 1,21 par du KBr solide sont déposées au fond d'un tube pour ultra-centrifugation puis recouvertes par des solutions de KBr (0,5ml) aux densités suivantes: 1,063-1,040-1,019-1,006. Les tubes sont ensuite ultracentrifugés à 50000g pendant 18h. Les BVSM-apoB flottent entre les densités 1,019 et 1,040 et cette plage de densité est du même ordre que celle des LDL. L'analyse des fractions du gradient en apoB et en radioactivité (coeur acylé marqué au ³H) montre que tous les BVSM-apoB se trouvent bien entre ces deux densités.

### -étude du métabolisme cellulaire des BVSM-apoB

Cette étude est réalisée sur des cellules-modèles, les fibroblastes de peau humaine.

### - par radiomarquage de l'apoB

Après radiomarquage de l'apoB par l'¹²⁵I, nous avons quantifié la fixation, l'internalisation et la dégradation des BVSM-apoB selon la technique de Brown et Golstein (J.Biol.Chem. (1974) 249,5153-5162).

Les BVSM-apoB (1mg d'apoB/1ml) sont mélangés à 0,25ml de tampon glycine pH 9,1 et à 10ul d'¹²⁵I (Ammersham). 31ul de solution d'lCl 1,25mM sont ajoutés et le mélange est ensuite agite doucement pendant 3min pour permettre l'oxydation des résidus tyrosine de l'apoB. Les différents produits sont séparés par perméation de gel sur colonne Pd10 (Sephadex G-25, Pharmacia) préalablement équilibrée par une solution de NaCl 0,9% et collectés par fraction de 1ml. Ces fractions sont ensuite dosées en protéines et en radioactivité. Celles qui contiennent les BVSM-apoB radiomarqués sont réunies puis dosées de nouveau. L'activité spécifique est de 175cpm/ng d'apoB.

Les fibroblastes (30 000cellules/puits) sont ensemencés dans des plaques de culture cellulaire a 6 puits (Nunc, diamètre 25mm) en milieu MEM (Gibco) contenant 10% de sérum de veau foetal (Gibco) pendant 48h puis conditionnées pendant 24h en milieu MEM contenant du sérum délipoprotéiné (MEM-LPDS). Pour chaque puits, le milieu est ensuite remplacé par 1ml de MEM-LPDS contenant des quantités croissantes de BVSM-apoB radiomarqués (5 à 25ug d'apoB). En parallèle, une série de puits contenant les mêmes quantités de BVSM-apoB radiomarqués et 40 fois plus de LDL non-radiomarquées est réalisée pour déterminer la fixation, l'internalisation et la dégradation non-spécifique des BVSM-apoB par les fibroblastes. Les cellules sont ensuite incubées pendant 5h à 37°C sous atmosphère contrôlée à 5% de CO₂.

La dégradation est quantifiée par la quantité d'¹²⁵I-tyrosine/cellule (cpm/mg de protéine) relarguée dans le milieu de culture, la fixation par la quantité d'¹²⁵I/cellule (cpm/mg de protéines) libérée après clivage des liaisons apoB-récepteur par l'héparine et l'internalisation par la quantité d'¹²⁵I/cellule (cpm/mg de protéines) libéré après lyse des cellules.

Ces expériences montrent que les BVSM-apoB suivent le même devenir cellulaire que les LDL naturelles et présentent la même affinité cellulaire que celles-ci. Ces résultats sont confirmés par des expériences d'immunofluorescence indirecte et d'ultrastructure en microscopie électronique.

### - par immunofluorescence indirecte

Les fibroblastes sont cultivés en monocouche sur des lamelles de verre placées dans des puits de culture comme précédemment et incubées avec les mêmes quantités de BVSM-apoB pendant 1h à 4°C pour la fixation et 15 à 30min à 37°C pour l'internalisation.

Les cellules sont ensuite lavées par du PBS et fixées avec de la paraformaldéhyde 4% dans du PBS pendant 15min à température ambiante. Les groupements aldéhydes libres restants sont réduits par du borohydrure de sodium. Les cellules sont perméabilisées avec du triton-X100 à 0,05 ou 1% dans du PBS à température ambiante puis lavées 1 fois avec du PBS et 2 fois avec du PBS + 1% BSA. Elles sont ensuite incubées pendant 1h à 37°C en présence de 150ul de solutions d'anticorps polyclonal anti-apoB de chèvre (Immuno France) diluées au 1/10^{ième} puis lavées au PBS + 1% BSA pour éliminer l'anticorps non-fixé. Le complexe apoB-anticorps ainsi formé est ensuite révélé par un anticorps secondaire anti-IgG de chèvre couplé à l'isothiocyanate de fluoroscéine (RAG-FITC, Tebu) par incubation des cellules à 37°C pendant 30min avec la solution d'anticorps secondaire diluée au 1/40^{ième}.

Les cellules sont ensuite observées au microscope à fluorescence (Ortholux-II Leitz). Les BVSM-apoB internalisés sont mises en évidence par les plages de fluorescence observées à l'intérieur des cellules.

### - par microscopie électronique de transmission

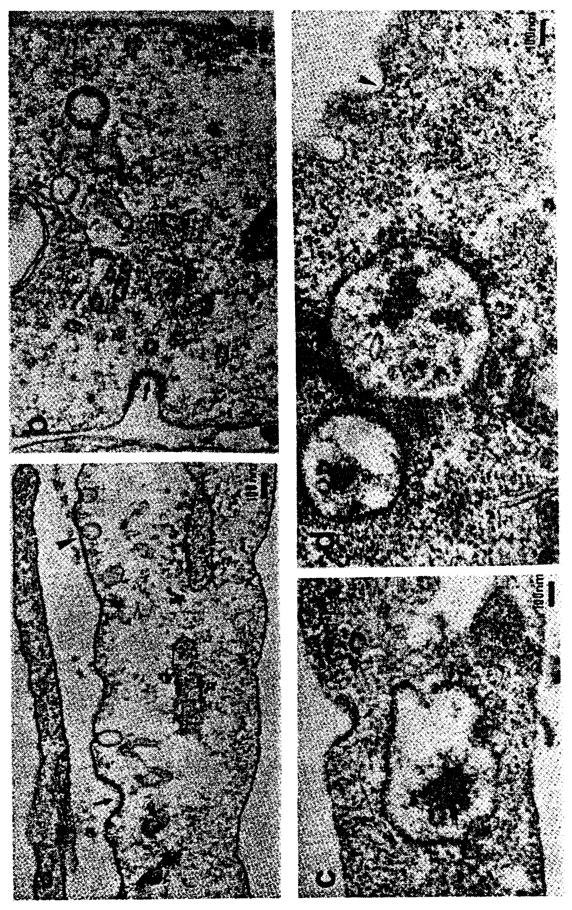

La fixation aux récepteurs cellulaires et l'internalisation des BVSM-apoB sont observées en microscopie électronique de transmission soit par inclusion des cellules dans de la résine soit par révélation des sucres par la méthode de Thierry comme précédemment. Cette technique visualise les différentes étapes du métabolisme : fixation des BVSM-apoB aux récepteurs, formation de puits recouverts, endocytose des BVSM-apoB dans des endosomes et dégradation par le lysosome à l'intérieur de la cellule. (cf figure 3).

Après lavage au PBS 0,1 M, les cellules sont fixées pendant 1h à 0-4°C avec une solution de glutaraldéhyde 3% dans du tampon cacodylate de sodium 0,1M, pH= 7,2. Les monocouches sont alors rincées avec le tampon cacodylate de sodium puis post-fixées 1h à 0-4°C par du tétroxyde d'osmium 1%. Les cellules sont lavées de nouveau avec du tampon cacacodylate de sodium puis déshydratées par des bains successifs d'éthanol de 50, 70, 100%. Lors du passage dans l'éthanol à 70%, les cellules sont délicatement détachées de la boite par grattage puis récupérées après 3min de centrifugation à 12000trs/min. Les deux derniers bains d'éthanol sont effectués sur les culots cellulaires. Ces échantillons sont ensuite imprégnés dans un mélange éthanol-Epon pur. La polymérisation de la résine est effectuée à 60°C pendant 72h. Les coupes ultrafines sont réalisées avec des couteaux de verre en utilisant un ultra-microtome.

Les coupes déposées sur des grilles de cuivre sont contrastées par une solution saturée d'acétate d'uranyle ( 20min à température ambiante), rincées à l'eau distillée puis par du citrate de plomb en atmosphère sèche. Après plusieurs rinçages à l'eau, les cellules sont observées au microscope électronique.

Les micro graphies montrent les différentes étapes du métabolisme cellulaire des BVSM-apoB qui est identique à celui des LDL.

### EXEMPLE 20 : CARACTERISATION DES BVSM-apoB CHARGES EN DOXORUBICINE

On utilise les BVSM chargés en doxorubicine préparés selon l'exemple 12 b). Le greffage de l'apoB est réalisé selon la méthode de dialyse de détergent décrite dans l'exemple 5.

### - perméation de gel

Les BVSM-apoB sont analysés par perméation de gel en FPLC sur colonne Superose 6 avec une double détection en spectrométrie à 280nm pour l'apoB et à 496nm pour la doxorubicine. Les profils chromatographiques obtenus montrent que l'apoB et la doxorubicine sont coéluées dans les mêmes fractions, correspondantes aux LDL et aux BVSM, et donc sont associées aux BVSM.

### - cytofluorimétrie de flux

Après 48h de culture en milieu délipoprotéiné, les fibroblastes humains normaux sont incubés avec de la doxorubicine sous forme libre ou encapsulée dans des BVSM ou des BVSM-apoB. Après incubation, de 0, à 2h, ils sont lavés, mis en contact avec de la trypsine 0,05% pendant 1min à37°C, repris en suspension dans du milieu de culture et fixés par du paraformaldéhyde. Les suspensions cellulaires obtenues sont analysées à l'aide d'un cytofluorimètre de flux (Becton-Dickinson Fac-scan) et l'incorporation cellulaire de la doxorubicine est mesurée par l'intensité de fluorescence émise. Les témoins fibroblastes avec BVSM et BVSM-apoB sans doxorubicine ne présentent aucune fluoresence.

Les résultats obtenus sont présentés dans le tableau suivant :

| Intensité de fluorescence émise | | | |
|---|---|---|---|
| Temps d'incubation (min) | Doxorubicine libre | Doxorubicine BVSM | Doxorubicine BVSM-apoB |
| 0 | 5 | 5 | 5 |
| 10 | 22 | 25 | 63 |
| 30 | 25 | 38 | 75 |
| 60 | 31 | 57 | 95 |
| 120 | 42 | 66 | 116 |

Les résultats présentés dans le tableau précédent indiquent que, quel que soit le temps d'incubation, l'internalisation de la doxorubicine encapsulée dans les BVSM-apoB est nettement plus importante que celle de la doxorubicine libre et de la doxorubicine encapsulée dans les BVSM. Les résultats sont en faveur d'une internalisation active des BVSM-apoB par les voies du récepteur des LDL.

### - test de cytotoxicité MTT

L'effet cytotoxique de la doxorubicine sous forme libre, de BVSM ou de BSVM-apoB, est évalué par un test qui mesure la viabilité cellulaire, le test au MTT. Celui-ci est basé sur la capacité vitale des cellules à réduire un dérivé du tétrazolium, le MTT, en formazan, par les déshydrogénases mitochondriales présentes uniquement dans les cellules vivantes et actives métaboliquement. Le formazan, après dilution dans le DMSO, est dosé par colorimétrie à 570nm.

Les tests de cytotoxicité sont réalisés sur une lignée tumorale d'adénocarcinome pulmonaire A549 qui possède un récepteur aux LDL fonctionnel.

On observe que, pour une concentration de 2µM, le pourcentage de cellules vivantes est de 13% pour la doxorubicine sous forme de BVSM-apoB et de 20% pour la doxorubicine sous forme de BVSM. Lorsqu'on ajoute un excès de LDL (75 fois plus en apoB que les BVSM-apoB) aux suspensions cellulaires en même temps que la doxorubicine, dans les BVSM ou les BVSM-apoB, à 2uM, on observe que le pourcentage de cellules vivantes est de 28% pour la doxorubicine dans les BVSM-apoB alors qu'aucun changement n'apparaît pour la doxorubicine dans les BVSM.

Des témoins ont été réalisés en parallèle et ont mis en évidence que la doxorubicine est bien encapsulée de manière stable à l'intérieur des BVSM, avec et sans apoB, et qu'elle n'est pas libérée après incubation dans les milieux utilisés pour la culture cellulaire. L'absence de cytotoxicité des seuls BVSM, avec ou sans apoB, a en outre été vérifiée.

Ces résultats montrent que la doxorubicine incorporée dans les BVSM-apoB conserve sa capacité d'être cytotoxique sur la lignée considérée. Après son passage dans les lysosomes, voie de pénétration intracellulaire obligée lorsque la doxorubicine est vectorisée par la voie du récepteur à apoB, la doxorubicine n'est pas détruite par les enzymes lysosomiales. Les expériences de compétition avec les LDL mettent en évidence que les BSM-apoB sont déplacés par le ligand naturel du récepteur à apoB et donc que la doxorubicine incorporée dans les BVSM-apoB est bien pilotée par l'apoB, greffée à la surface du BVSM.

## Revendications

1. Vecteur particulaire synthétique, caractérisé en ce qu'il possède un tropisme sélectif pour un type de cellules et en ce qu'il comporte :
- un noyau hydrophile non liquide,
- une première couche ou couronne de nature lipidique liée au noyau par des liaisons covalentes,
- une seconde couche ou feuillet externe de phospholipides liée à la première couche par des interactions hydrophobes,
- des molécules d'apolipoprotéine B ou de ligands protéiques ou peptidiques capables de reconnaître spécifiquement les récepteurs cellulaires des LDL, greffées sur la couche de phospholipides.

2. Vecteur particulaire selon la revendication 1, caractérisé en ce qu'il possède un tropisme sélectif pour les cellules tumorales.

3. Vecteur particulaire selon la revendication 1, caractérisé en ce que les molécules d'apolipoprotéine B sont modifiées.

4. Vecteur particulaire selon la revendication 3, caractérisé en ce qu'il possède un tropisme sélectif pour les macrophages.

5. Vecteur particulaire selon l'une des revendications 1 à 4, caractérisé en ce que le noyau hydrophile est constitué d'un polysaccharide réticulé.

6. Vecteur particulaire selon l'une des revendication 1 à 5, caractérisé en ce que le polysaccharide est choisi dans le groupe comprenant le dextrane, l'amidon et la cellulose et leurs dérivés.

7. Vecteur particulaire selon l'une des revendications 1 à 6, caractérisé en ce que la première couche de nature lipidique est fixée par acylation régiosélective d'acides gras à la périphérie d'un noyau de polysaccharides réticulés.

8. Vecteur particulaire selon l'une des revendications 1 à 7, caractérisé en ce que le noyau hydrophile comporte en outre un principe actif.

9. Vecteur particulaire selon la revendication 8, caractérisé en ce que le principe actif est choisi dans le groupe comprenant des médicaments anticancéreux, réversants de résistances aux médicaments anticancéreux, immunomodulateurs, oligonucléotides, ARN- messagers antisens, hépatoprotecteurs, inhibiteurs de HMGCoAréductase, peptide de régulation cellulaire.

10. Vecteur particulaire selon la revendication 8, caractérisé en ce que le principe actif est un agent anticancéreux choisi parmi les molécules suivantes : Aclarubicine, Epirubicine, Daunorubicine, Doxorubicine, Zorubicine.

11. Vecteur particulaire selon la revendication 8, caractérisé en ce que le principe actif est choisi dans le groupe comprenant antiviraux, antibactériens, antifongiques, antiparasitaires, antiSida.

12. Vecteur particulaire selon l'une des revendications 1 à 11, caractérisé en ce qu'il a une dimension d'environ 20nm.

13. Procédé de préparation d'un vecteur selon l'une des revendications 1 à 12, caractérise en ce que :
a)on prépare des vecteurs comportant un noyau hydrophile, une première couche de lipides et une deuxième couche de phospholipides,
b)on encapsule un principe actif à l'intérieur du noyau hydrophile,
c)on greffe sur la couche phospholipidique des molécules d'apolipoprotéine B purifiée à partir de LDL ou de ligands protéiques ou peptidiques capablent de reconnaître spécifiquement les récepteurs cellulaires des LDL.

14. Procédé selon la revendication 13, caractérisé en ce que le greffage de l'étape c) est réalisé par la technique de dialyse de détergent à partir d'apolipoprotéine B solubilisée sous forme de micelles.

15. Procédé selon l'une des revendications 13 et 14, caractérisé en ce que le détergent utilisé est choisi parmi les détergents dialysables.

16. Composition pharmaceutique, caractérisée en ce qu'elle comporte un vecteur particulaire selon l'une des revendications 1 à 12 et un support acceptable pour son administration.

## Claims

1. Synthetic particulate vector, characterized in that it possesses a selective tropism for one cell type and in that it contains :
- a non-liquid hydrophilic core,
- a first layer or shell of lipid nature linked to the core via covalent bonds,
- a second layer or outer lamella of phospholipids, linked to the first layer via hydrophobic interactions,
- molecules of apolipoprotein B, or of protein or peptide ligands capable of specifically recognizing cellular LDL receptors, grafted onto the layer of phospholipids.

2. Particulate vector according to claim 1, characterized in that it possesses a selective tropism for tumour cells.

3. Particulate vector according to claim 1, characterized in that the molecules of apolipoprotein B are modified.

4. Particulate vector according to claim 3, characterized in that it possesses a selective tropism for macrophages.

5. Particulate vector according to one of claims 1 to 4, characterized in that the hydrophilic core consists of crosslinked polysaccharide.

6. Particulate vector according to one of claims 1 to 5, characterized in that the polysaccharide is chosen from the group comprising dextran, starch and cellulose and their derivatives.

7. Particulate vector according to one of claims 1 to 6, characterized in that the first layer of lipid nature is bound by regioselective acylation of fatty acids at the periphery of a crosslinked polysaccharide core.

8. Particulate vector according to one of claims 1 to 7, characterized in that the hydrophilic core contains, in addition, an active principle.

9. Particulate vector according to claim 8, characterized in that the active principle is chosen from the group comprising anticancer drugs, agents that reverse resistance to anticancer drugs, immunomodulators, oligonucleotides, antisense messenger RNAs, hepatoprotective agents, HMGCoA reductase inhibitors and cell-regulation peptide.

10. Particulate vector according to claim 8, characterized in that the active principle is an anticancer agent chosen from the following molecules : aclarubicin, epirubicin, daunorubicin, doxorubicin, zorubicin.

11. Particulate vector according to claim 8, characterized in that the active principle is chosen from the group comprising antiviral, antibacterial, antifungal, antiparasitic and anti-AIDS drugs.

12. Particulate vector according to one of claims 1 to 11, characterized in that it is approximately 20 nm in size.

13. Method for preparing a vector according to one of claims 1 to 12, characterized in that :
a) vectors containing a hydrophilic core, a first layer of lipids and a second layer of phospholipids are prepared,
b) an active principle is encapsulated within the hydrophilic core,
c) molecules of apolipoprotein B purified from LDL, or of protein or peptide ligands capable of specifically recognizing cellular LDL receptors, are grafted onto the phospholipid layer.

14. Method according to claim 13, characterized in that the grafting in step c) is carried out by the detergent dialysis technique starting with apolipoprotein B solubilized in the form of micelles.

15. Method according to one of claims 13 and 14, characterized in that the detergent used is chosen from dialysable detergents.

16. Pharmaceutical composition, characterized in that it contains a particulate vector according to one of claims 1 to 12 and a vehicle which is acceptable for its administration.

## Patentansprüche

1. Synthetischer teilchenförmiger Vektor, dadurch gekennzeichnet, daß er einen für einen Zellentyp selektiven Tropismus aufweist und umfaßt:
- einen nicht flüssigen hydrophilen Kern,
- eine erste Schicht oder Krone (Kranz) von Lipid-Natur, die über kovalente Bindungen an den Kern gebunden ist,
- eine zweite äußere Schicht oder Lamelle aus Phospholipiden, die durch hydrophobe Wechselwirkungen an die erste Schicht gebunden ist, und
- Apolipoprotein B-Moleküle oder Protein- oder Peptid-Liganden, die in der Lage sind, die Zellrezeptoren der LDL spezifisch zu erkennen, die auf die Phospholipid-Schicht aufgepfropft sind.

2. Teilchenförmiger Vektor nach Anspruch 1, dadurch gekennzeichnet, daß er einen für Tumorzellen selektiven Tropismus aufweist.

3. Teilchenförmiger Vektor nach Anspruch 1, dadurch gekennzeichnet, daß die Apolipoprotein B-Moleküle modifiziert sind.

4. Teilchenförmiger Vektor nach Anspruch 3, dadurch gekennzeichnet, daß er einen für Makrophagen selektiven Tropismus aufweist.

5. Teilchenförmiger Vektor nach einem der Ansprüche 1 bis 4, dadurch gekennzeichnet, daß der hydrophile Kern aus einem vernetzten Polysaccharid besteht.

6. Teilchenförmiger Vektor nach einem der Ansprüche 1 bis 5, dadurch gekennzeichnet, daß das Polysaccharid ausgewählt wird aus der Gruppe, die Dextran, Stärke und Cellulose sowie ihre Derivate umfaßt.

7. Teilchenförmiger Vektor nach einem der Ansprüche 1 bis 6, dadurch gekennzeichnet, daß die erste Schicht von Lipid-Natur durch regioselektive Fettsäure-Acylierung an den Umfang eines Kerns von vernetzten Polysacchariden gebunden ist.

8. Teilchenförmiger Vektor nach einem der Ansprüche 1 bis 7, dadurch gekennzeichnet, daß der hydrophile Kern außerdem einen Wirkstoff (aktives Prinzip) enthält.

9. Teilchenförmiger Vektor nach Anspruch 8, dadurch gekennzeichnet, daß der Wirkstoff (das aktive Prinzip) ausgewählt wird aus der Gruppe, die umfaßt Antikrebs-Arzneimittel, Agentien, welche die Resistenz gegen Antikrebs-Arzneimittel umkehren, Immunmodulatoren, Oligonucleotide, nichtcodierende ("anti-sens") RNA-Botenstoffe, Hepatoprotektoren, HMGCoA-Reduktase-Inhibitoren und Zellregulations-Peptid.

10. Teilchenförmiger Vektor nach Anspruch 8, dadurch gekennzeichnet, daß der Wirkstoff (das aktive Prinzip) ein Antikrebsmittel ist, das ausgewählt wird aus den folgenden Molekülen: Aclarubicin, Epirubicin, Daunorubicin, Doxorubicin und Zorubicin.

11. Teilchenförmiger Vektor nach Anspruch 8, dadurch gekennzeichnet, daß der Wirkstoff (das aktive Prinzip) ausgewählt wird aus der Gruppe, die Antiviren-, Antibakterien-, Antifungi-, Antiparasiten- und Antiaids-Mittel umfaßt.

12. Teilchenförmiger Vektor nach einem der Ansprüche 1 bis 11, dadurch gekennzeichnet, der er eine Dimension von etwa 20 nm hat.

13. Verfahren zur Herstellung eines Vektors nach einem der Ansprüche 1 bis 12, dadurch gekennzeichnet, daß man
a) Vektoren herstellt, die einen hydrophilen Kern, eine erste Lipid-Schicht und eine zweite Phospholipid-Schicht umfassen,
b) einen Wirkstoff (ein aktives Prinzip) im Innern des hydrophilen Kerns einkapselt und
c) auf die Phospholipid-Schicht Apolipoprotein B-Moleküle, die ausgehend von LDL, gereinigt worden sind, oder Protein- oder Peptid-Liganden aufpfropft, die in der Lage sind, die Zellrezeptoren der LDL spezifisch zu erkennen.

14. Verfahren nach Anspruch 13, dadurch gekennzeichnet, daß das Aufpfropfen in der Stufe (c) unter Anwendung der Detergens-Dialyse-Methode durchgeführt wird, wobei man von Apolipoprotein B ausgeht, das in Form von Micellen solubilisiert worden ist.

15. Verfahren nach einem der Ansprüche 13 und 14, dadurch gekennzeichnet, daß das verwendete Detergens unter den dialysierbaren Detergentien ausgewählt wird.

16. Pharmazeutische Zusammensetzung, dadurch gekennzeichnet, daß sie einen Partiular-Vektor nach einem der Ansprüche 1 bis 12 und einen für ihre Verabreichung akzeptablen Träger umfaßt.
